(19) Europäisches Patentamt
European Patent Office
Office européen des brevets

(11) Numéro de publication: **0 430 738 A2**

(12) **DEMANDE DE BREVET EUROPEEN**

(21) Numéro de dépôt: **90403156.4**

(51) Int. Cl.$^5$: **A61K 31/44**

(22) Date de dépôt: **07.11.90**

(30) Priorité: **30.11.89 FR 8915768**

(43) Date de publication de la demande: **05.06.91 Bulletin 91/23**

(84) Etats contractants désignés: **AT BE CH DE DK FR GB IT LI LU NL SE Bulletin**

(71) Demandeur: **SYNTHELABO**
**58 rue de la Glacière**
**F-75013 Paris(FR)**

(72) Inventeur: **Depoortere, Henri**
**5, Résidence des Graviers, Route de Limours**
**Cernay La Ville, F-78720 Dampierre(FR)**
Inventeur: **George, Pascal**
**19, Rue des Quatre Vents**
**F-78730 St. Arnoult en Yvelines(FR)**

(74) Mandataire: **Thouret-Lemaitre, Elisabeth et al**
**SYNTHELABO, 22, avenue Galilée, B.P. 72**
**F-92350 Le Plessis-Robinson Cédex(FR)**

# (54) Utilisation d'une imidazopyridine pour la fabrication de médicaments anesthésiques.

(57) Utilisation du N-[[(éthyl-4 phényl)-2 imidazo[1,2-a] pyridinyl-3]méthyl] N,3-diméthyl-butanamide et de ses sels pharmaceutiquement acceptables pour la fabrication de médicaments anesthésiques.

EP 0 430 738 A2

La présente invention a pour objet l'utilisation da N-[[(éthyl-4 phényl)-2 imidazo[1,2-a]pyridinyl-3]-méthyl] N,3-diméthyl-butanamide, ou de ses sels d'addition à des acides pharmaceutiquement acceptables, pour la fabrication de médicaments anesthésiques.

Le N-[[(éthyl-4 phényl)-2 imidazo[1,2-a]-pyridinyl-3]méthyl] N,3-diméthyl-butanamide de formule

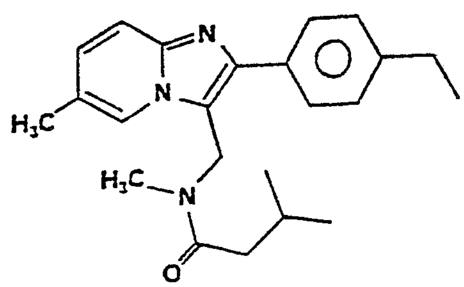

et ses sels d'addition à des acides pharmaceutiquement acceptables ont été décrits dans le brevet européen n° 0172096.

La préparation du composé et de son méthanesulfonate est décrite ci-après et illustrée dans le schéma réactionnel.

1. Préparation du N-[[(éthyl-4 phényl)-2 imidazo-[1,2-a] pyridinyl-3]méthyl] N,3-diméthyl-butanamide.

1.1. Bromo-2 (éthyl-4 phényl)-1 éthanone-1.

A une solution de 40 g (40 ml) (0,27 mole) de (éthyl-4 phényl)-1 éthanone-1 dans 250 ml de $CH_2Cl_2$, on ajoute goutte à goutte, 43,7 g (14 ml) (0,27 mole) de brome. L'addition terminée, le mélange est agité 1/2 h puis lavé plusieurs fois à l'eau. La phase organique est décantée, séchée sur $MgSO_4$, filtrée, le solvant évaporé : on obtient 60 g d'huile brune. Le produit est utilisé tel quel pour l'étape suivante.

1.2. Méthyl-6 (éthyl-4 phényl)-2 imidazo[1,2-a]-pyridine.

Dans un erlenmeyer de 1 l, on introduit successivement : 60 g (0,2 mole) de bromo-2 (éthyl-4 phényl)-1 éthanone-1 préparée à l'étape précédente (pureté 77 %), 21,7 g (0,2 mole) de amino-2 méthyl-5 pyridine, 34 g (0,4 mole) de $NaHCO_3$ et 540 ml d'éthanol à 95 %.

Le mélange est porté à reflux 1 h 30, puis le solvant est évaporé. Le résidu est repris au $CH_2Cl_2$ et lavé à l'eau. La phase organique est décantée, séchée sur $Na_2SO_4$, filtrée et le solvant évaporé. Le résidu est repris à l'éther, on obtient un solide que l'on sèche sous vide.

Rendement : 46 g soit 100 %. F = 146-147° C.

1.3. Méthyl-6 (éthyl-4 phényl)-2 imidazo[1,2-a]-pyridine-3-méthanol.

15 g (0,0634 mole) de l'imidazopyridine précédente et 47,6 g (44 ml) (0,587 mole) de formol à 37 % dans l'eau sont introduits dans 225 l d'acide acétique glacial. La solution est portée à 50° C et maintenue sous agitation 3 h. L'acide et l'eau sont ensuite évaporés. Le résidu est repris à l'eau et l'ammoniaque jusqu'à pH ≧ 9 ainsi que par $CH_2Cl_2$. L'insoluble entre les deux phases est collecté par filtration et séché.

Rendement : 7,37 g soit 43 %. F = 215-216° C.

1.4. N-[[(éthyl-4 phényl)-2 imidazo[1,2-a]pyridinyl-3]méthyl]3-méthyl-butanamide.

2,4 g (0,009 mole) d'imidazo[1,2-a]pyridine-3-méthanol et 24 ml d'isovaléronitrile sont introduits dans un ballon. Le mélange est agité. On y ajoute goutte-à-goutte, 4,64 g (2,52 ml) (0,0453 mole) d'acide sulfurique. Le mélange est chauffé jusqu'à ce qu'une huile décante. Le liquide surnageant est utilisé pour une seconde fraction. L'huile (le culot) est hydrolysée à la glace puis traitée à l'ammoniaque. L'amide est extrait au $CH_2Cl_2$, le solvant évaporé et le résidu lavé au pentane puis ensuite séché.

Rendement : 8,4 g soit 89 %. F = 154-155° C.

1.5. N-[[(éthyl-4 phényl)-2 imidazo[1,2-a]pyridinyl-3]méthyl] N,3-diméthyl-butanamide.

Dans un ballon de 250 ml on introduit 1,28 g (0,0268 mole) de NaH à 50 % dans l'huile et lave le mélange au pentane. On ajoute ensuite 33 ml de THF et 1,7 ml de DMF et purge à l'argon. On ajoute 4,68 g (0,134 mole) d'amide secondaire dissous dans 55 ml de THF et 2,7 ml de DMF contenant 1,7 ml (0,268 mole) de $CH_3I$. Le mélange est agité à la température ambiante 1 h. En fin de réaction, l'excès de NaH est détruit par un peu de méthanol et le solvant est évaporé. Le résidu est repris au $CH_2Cl_2$ et la phase organique lavée, décantée puis séchée sur $Na_2SO_4$ Après filtration, le solvant est évaporé. Le résidu est purifié par chromatographie flash. ($CH_2Cl_2$ 97-$CH_3OH$ 3).

Rendement : 3,58 g soit 73 %.

2. Préparation du méthanesulfonate du N-[[(éthyl-4 phényl)-2 imidazo[1,2-a]pyridinyl-3]méthyl] N-3-diméthyl-butanamide.

On prépare le méthanesulfonate à partir de 4 g (0,0110 mole) de base et de 1,05 g (0,0110 mole)

d'acide méthanesulfonique dans de l'éthanol. La solution est évaporée à sec et le résidu est repris à l'éther, puis recristallisé dans de l'acétate d'éthyle. Rendement : 4,2 g (83 %). F = 136-138 °C.

L'activité anesthésique du composé sous forme de méthanesulfonate a été déterminée par observation de l'action du composé sur l'ECoG du rat immobilisé (Depoortere, H. et al. Neuropsychobiology (1986) 16,157-162). Chez le rat immobilisé le produit à étudier a été injecté par voie intraveineuse aux doses croissantes de 0,003 mg/kg à 30 mg/kg. Il induit des tracés de sommeil à partir de la dose de 0,003 mg/kg i.v.

Aux doses de 3 à 10 mg/kg i.v., le produit induit des tracés caractéristiques d'un anesthésique général (rythme d'ondes monomorphes prédominant de 3Hz également observé après 30 mg/kg i.v. de midazolam). A la dose de 30 mg/kg i.v., les tracés présentent une activité isoélectrique entrecoupée de pointes "complexes K". Chez le rat, cette activité s'observe également après la kétamine et le propofol).

L'effet anesthésique général de courte durée d'action se caractérise sur le plan comportemental par la perte de réflexes sensitifs (durant environ 15 mn) après 5 mg/kg i.v. et du réflexe de retournement (durant 30 à 50 mn) après 5 mg/kg i.v. de composé.
L'anesthésie peut également être obtenue par une administration intrapéritonéale et/ou intramusculaire du produit.

Le produit est également doté d'une activité analgésique mise en évidence chez la souris sur le "writhing test" ($DA_{50}$ = 0,4 mg/kg i.v.) et sur le test de la plaque chauffante ($DA_{100}$ = 0,3 mg/kg i.v.).

Le composé et ses sels d'addition à des acides pharmaceutiquement acceptables peuvent être utilisés comme anesthésiques généraux pour l'induction et/ou le maintien de l'anesthésie. Ils peuvent également être utilisés dans des perfusions dans le cas de soins intensifs et aussi pour des interventions chirurgicales de courte durée (en dentisterie, endoscopies, etc...).
Le composé et ses sels d'addition à des acides pharmaceutiquement acceptables peuvent également être utilisés comme anesthésiques locaux, par exemple sous la forme de solutions de 0,25 à 2 % adrénalinées.

Le composé et ses sels peuvent être utilisés seuls ou en association avec d'autres anesthésiques (volatils ou non), des myorelaxants et/ou des analgésiques.
Ils peuvent être présentés sous toute forme d'administration appropriée (comprimés, gélules ou solutions injectables). La posologie peut aller de 0,01 à 0,3 mg/kg par voie intraveineuse.

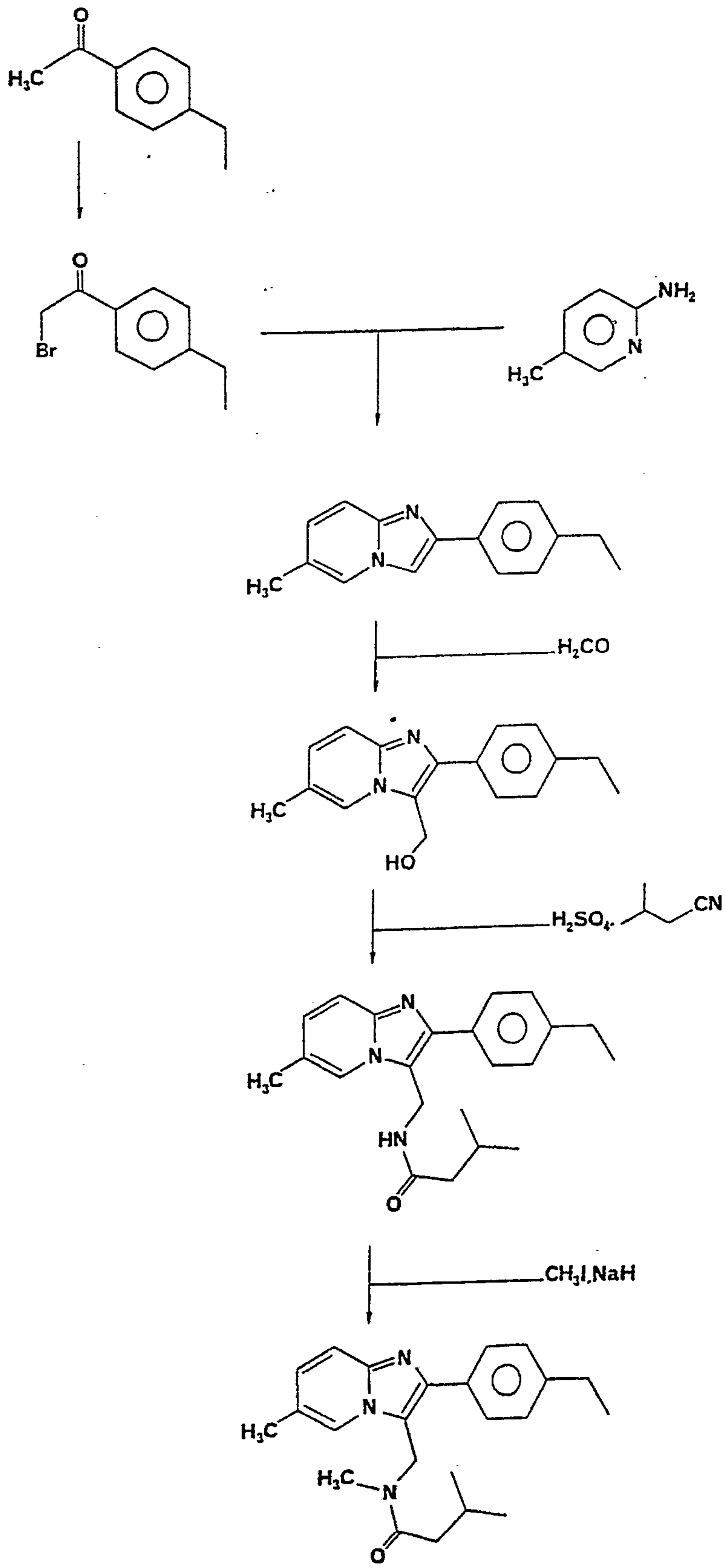


I

II

III

IV

V

VI

VII

Revendications pour les Etats contractants : AT, BE, CH, DE, DK, FR, GB, IT, LI, LU, NL, SE.

**Revendications**

1. Utilisation du N-[[(éthyl-4 phényl)-2 imidazo[1,2-a] pyridinyl-3]méthyl] N,3-diméthyl-butanamide et de ses sels pharmaceutiquement acceptables pour la fabrication de médicaments anesthésiques.

2. Utilisation du méthanesulfonate du N-[[(éthyl-4 phényl)-2 imidazo[1,2-a]pyridinyl-3]méthyl]-N,3-diméthyl-butanamide pour la fabrication de médicaments anesthésiques.